# DEMANDE DE BREVET EUROPEEN

(11) **EP 1 319 413 A1**
(43) Date de publication de la demande: **18.06.2003**
(21) Numéro de dépôt: 01403235.3
(22) Date de dépôt: 13.12.2001
(51) Int. Cl.: A61L 9/12

(54) **Dispositif de diffusion dans l'air ambiant et procédé de diffusion de produits**

(71) Demandeur: Poisson-Quinton, Pierre, 75011 Paris (FR)
(72) Inventeur: Poisson-Quinton, Pierre, 75011 Paris (FR)
(74) Mandataire: Rémont, Claude

(57) **Abrégé**

Le dispositif de diffusion (2) selon l'invention comprend un moyen de stockage (28, 30, 32) du premier produit à l'abri de l'air, des moyens de prélèvement (24) du premier produit à partir du dit moyen de stockage (28, 30, 32), un moyen de diffusion (10, 16) du premier produit prélevé et une unité centrale de traitement raccordée aux dits moyens de prélèvement (24) du premier produit et au dit moyen de diffusion (10, 16) du premier produit pour piloter ces derniers en fonction d'ordres de diffusion du premier produit. Il comporte au moins un deuxième moyen de stockage (28, 30, 32) d'au moins un deuxième produit de nature différente du premier produit, ledit deuxième moyen de stockage (28, 30, 32) communiquant avec lesdits moyens de prélèvement (24), et au moins un deuxième moyen de diffusion (10, 16), chacun des moyens de diffusion (10, 16) étant adapté à la nature du produit à diffuser.

## Description

La présente invention concerne un dispositif de diffusion dans l'air ambiant de produits et vise également un procédé de diffusion dans l'air ambiant de produits ou de compositions.

Une application particulière de ce type de dispositif est, par exemple, la diffusion dans l'air ambiant de produits, ayant des propriétés olfactives, comme par exemple des parfums, en synchronisme avec des images présentées sur un écran, ou d'une manière plus générale, sous le contrôle d'ordres de diffusion.

Dans ce type d'applications, pour la diffusion de produits, on utilise généralement un dispositif du type comprenant un moyen de stockage d'un premier produit à l'abri de l'air, des moyens de prélèvement du premier produit à partir du dit moyen de stockage, un moyen de diffusion du premier produit prélevé et une unité centrale de traitement raccordée aux dits moyens de prélèvement du premier produit et au dit moyen de diffusion du premier produit pour piloter ces derniers en fonction d'ordres de diffusion du premier produit.

Ce type de dispositif, bien que relativement efficace pour commander à distance ou localement la diffusion de produits, et ce, avec un temps de réponse relativement faible, présente un inconvénient majeur, relatif au fait que le nombre de produits susceptibles d'être diffusées est limité, dans la mesure où, d'une part, les moyens de stockage ne sont généralement pas adaptés pour recevoir différents types de produits et, d'autre part, le moyen de diffusion doit être adapté à la nature du produit à diffuser.

En particulier, les dispositifs de diffusion actuels sont généralement uniquement conçus pour le stockage et la diffusion de produits de même nature, en particulier de produits olfactifs de même nature.

On entend par produits de même nature, des produits pour lesquels un même moyen de diffusion, en termes de structure, est envisageable.

Ainsi, les dispositifs de diffusion actuels ne permettent pas de diffuser à la fois - c'est à dire soit de façon simultanée, soit de façon successive - des produits qui ne sont pas de même nature, par exemple des produits olfactifs élaborés à base d'extrait de matière naturelle tels que des huiles essentielles et des compositions à base d'eau ou d'alcool, ou de diffuser des compositions olfactives constituées par au moins deux produits.

Le but de l'invention est de pallier ces inconvénients.

A cet effet, conformément à l'invention, le dispositif de diffusion de produits est tel qu'il comporte au moins un deuxième moyen de stockage d'au moins un deuxième produit de nature différente du premier produit, ledit deuxième moyen de stockage communiquant avec lesdits moyens de prélèvement, et au moins un deuxième moyen de diffusion, structurellement distinct du premier moyen de diffusion, chacun des moyens de diffusion étant ainsi adapté à la nature du produit à diffuser.

On augmente alors considérablement les performances du dispositif en augmentant le nombre de produits différents susceptibles d'être diffusés, tout ou partie d'entre eux pouvant avoir un pouvoir olfactif.

Les produits à diffuser peuvent se trouver sous une forme liquide, solide ou gazeuse.

Selon une caractéristique de l'invention, l'un des moyens de diffusion est un pulvérisateur.

On peut ainsi diffuser tout produit susceptible d'être diffusé dans l'air ambiant selon ce mode de diffusion, en particulier des produits olfactifs tels que des parfums naturels ou de synthèse.

De préférence, l'un des moyens de diffusion est une chambre de diffusion qui communique avec l'air ambiant et dans laquelle débouchent les moyens de prélèvement.

Il est ainsi possible de diffuser un produit comme des huiles essentielles ou des éléments minéraux, mais également de diffuser une composition élaborée à partir d'un mélange d'au moins deux de ces produits de même nature.

Les premier et deuxième moyens de stockage comportent un ensemble de cellules servant chacune au stockage des premier et deuxième produits, et en ce que les moyens de prélèvement d'un des dits produits comportent un moyen de pompage.

De préférence, les cellules sont portées par des disques amovibles venant se monter sur une colonne délimitant intérieurement un conduit débouchant par l'une de ses extrémités dans le pulvérisateur et par son extrémité opposée dans la chambre de diffusion.

On connaît également un procédé de diffusion dans l'air ambiant d'un produit, à l'aide d'un dispositif comportant un moyen de stockage du premier produit à l'abri de l'air, et comprenant des étapes telles qu'on le prélève à partir du dit moyen de stockage, on le conduit dans un moyen de diffusion et on le diffuse, ces étapes étant pilotées et contrôlées par une unité centrale ou tout autre système de contrôle-commande.

Selon l'invention, ce procédé de diffusion du type précité est mis en oeuvre à l'aide d'un dispositif comportant au moins un deuxième moyen de stockage d'au moins un deuxième produit à diffuser de nature différente du premier produit, et au moins un deuxième moyen de diffusion, structurellement distinct du premier moyen de diffusion, chacun des moyens de diffusion étant adapté à la nature du produit à diffuser, et comprend en outre une étape de sélection du produit à diffuser et une étape de sélection du moyen de diffusion en fonction de la nature du produit à diffuser.

D'autres particularités de l'invention résulteront de la description qui va suivre, donnée à titre d'exemple non limitatif et faite en référence aux figures annexées dans lesquelles :
- la figure 1 est une vue schématique en coupe du dispositif selon l'invention muni de deux dispositifs de diffusion ;
- la figure 2 est une vue du dessus d'un moyen de stockage de produit ;
- la figure 3 illustre schématiquement un mode d'utilisation de dispositifs selon l'invention placés en réseau.

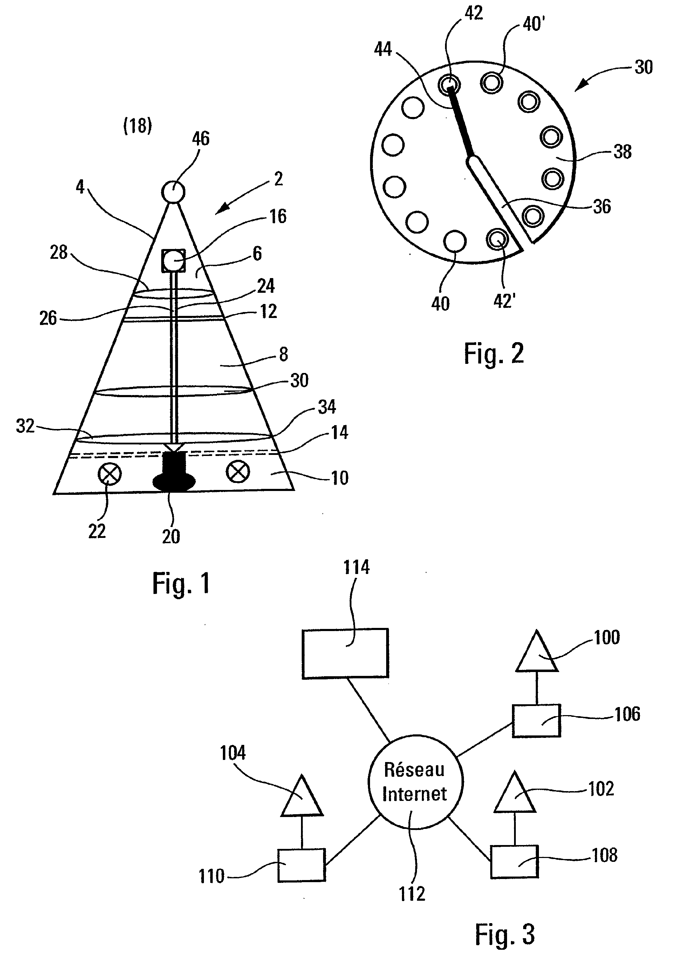

Le dispositif de diffusion 2 représenté à la figure 1 comporte un boîtier 4 de forme pyramidale et d'axe supposé vertical. Le boîtier 4 est divisé en trois chambres 6, 8 et 10 rendues hermétiquement étanches les unes des autres par la présence de membranes de séparation 12 et 14.

Une première chambre 6, située dans la partie supérieure du boîtier 4, comporte un pulvérisateur 16, dont l'orifice de diffusion (non représenté) est orienté vers l'extérieur du dispositif de diffusion 2, au niveau de l'air ambiant 18.

Une deuxième chambre 10, dite chambre de diffusion, située dans la partie inférieure du boîtier 4, est dotée intérieurement d'un réceptacle 20 dans lequel débouche les moyens de prélèvement, ce réceptacle 20 communiquant avec la deuxième chambre de diffusion 10. Cette dernière est également équipées de moyens de ventilation, tels que des ventilateurs 22, pilotés par l'unité centrale.

Une chambre intermédiaire 8, séparée des première et deuxième chambres 6 et 10, respectivement par les membranes de séparation 12 et 14, est située dans la partie intermédiaire du boîtier 4.

Les trois chambres 6, 8 et 10 sont traversées par une colonne 24 axiale s'étendant verticalement dans le boîtier 4.

La colonne 24 délimite intérieurement un conduit 26 qui débouche par l'une de ses extrémités, en l'occurrence son extrémité supérieure, dans le pulvérisateur 16 et par son extrémité opposée, en l'espèce inférieure, dans la deuxième chambre de diffusion 10.

La colonne 24 comporte également une pompe (non représentée) destinée à aspirer les produits à diffuser puis à les diriger soit vers le pulvérisateur 16, soit vers la deuxième chambre de diffusion 10.

Des disques 28, 30 et 32 amovibles viennent se monter sur la colonne 24.

De préférence, les disques 28, 30 et 32 sont de forme et de dimensions adaptées à la forme et aux dimensions du boîtier 4.

Un premier disque 28, disposé dans la première chambre 6, est muni de cellules (non représentées) qui contiennent les produits destinés à être diffusés dans l'air ambiant 18 par l'intermédiaire du pulvérisateur 16.

On peut ainsi envisager la diffusion de tout type de produits tels que, par exemple, des solutions à base d'alcool comme les parfums, que ces derniers soient à base d'essences naturelles ou de synthèse, ou à base d'eau. On peut de même envisager de diffuser des produits comprenant des oligo-éléments et/ou des sels minéraux dilués dans des solutions aqueuses.

Des deuxième et troisième disques 30 et 32, disposés dans la chambre intermédiaire 8, sont munis de cellules (non représentées) qui contiennent les produits destinés à être diffusés dans l'air ambiant 18 par l'intermédiaire de la deuxième chambre de diffusion 10.

Chacun des deuxième et troisième disques 30 et 32 est de préférence conçu pour contenir des produits de même nature, tels que des arômes, des huiles essentielles naturelles ou de synthèse,...

Compte tenu de cette distinction faite sur la nature des produits à diffuser et dans le but d'éviter tout mélange non désiré desdits produits lors de l'utilisation du dispositif 2 selon l'invention, la première chambre 6 est séparée de façon étanche de la chambre intermédiaire 8 par la membrane de séparation 12.

La membrane de séparation 12 est constituée d'éléments absorbants qui ne dénaturent pas les propriétés des produits contenus sur les premier, deuxième et troisième disques 28, 30 et 32.

Les premier, deuxième et troisième disques 28, 30 et 32 sont angulairement déplaçables par rapport à la colonne 24 sous l'action de moyens moteurs (non représentés).

Avantageusement, le dispositif de diffusion 2 selon l'invention est équipé d'un capteur 46 disposé, comme l'indique la figure 1, au sommet du boîtier 4.

Le capteur 46 est destiné à fournir des données sur l'air ambiant 18, ces données étant recueillies, analysées et traitées par l'unité centrale. Il permet ainsi de mesurer divers paramètres de l'air ambiant 18 dans lequel est placé le dispositif de diffusion 2 et, en particulier, le taux d'humidité ou d'ionisation, la température,...

Le capteur 46 ainsi que les moyens moteurs mentionnés ci-dessus sont reliés à une unité centrale qui comporte, stockés en mémoire, un programme de pilotage du dispositif de diffusion 2 ainsi que des données d'identification des premier, deuxième et troisième disques 28, 30 et 32 et/ou des cellules 42, 42' décrites ci-dessous. Ce programme de pilotage peut avantageusement piloter le capteur 46 et les moyens moteurs en fonction des informations délivrées par le capteur 46.

La figure 2 représente une vue du dessous de l'un des disques. Dans ce qui suit, on va décrire la structure du troisième disque 32 mais, bien entendu, cette description s'applique également aux premier et deuxième disques 28 et 30.

Le disque 32, d'un diamètre adapté au diamètre transversal du boîtier 4 au niveau de son ouverture 34, est muni d'une encoche 36 pratiquée sur un rayon du disque 32.

La largeur de l'encoche 36 correspond sensiblement au diamètre de la colonne 24 afin de permettre, lors de l'introduction latérale du disque 32 dans le boîtier 4 au niveau de son ouverture 34, le placement de la colonne 24 au centre du disque 32. L'encoche 36 sert ainsi de référence au positionnement angulaire du disque 32.

Le disque 32 peut être maintenu dans le boîtier 4 grâce à la présence de moyens de fixation amovibles, tel qu'un moyen d'encliquetage.

Au niveau du bord 38 du disque 32 sont pratiquées des ouvertures 40, 40' à l'intérieur desquelles sont disposées des cellules 42, 42' contenant chacune un produit destiné à être diffusé, selon le même mode de diffusion.

Le produit contenu dans chacune des cellules 42, 42' d'un même disque 32 est de préférence de même nature et peut être avantageusement de formulation distincte d'une cellule à l'autre.

De préférence, le disque 32 est divisé en compartiments (non représentés) hermétiquement isolés les uns des autres contenant chacun une cellule 42, 42'. Ces compartiments peuvent être fabriqués par moulage par injection.

Les cellules 42, 42' sont réalisées dans un matériau adapté à la nature du produit à contenir, par exemple en verre ou en matériau composite. Elles comportent chacune une embouchure obturée par une valve (non représentée), cette embouchure pouvant être apposée au moment de conditionnement du produit à diffuser dans sa cellule.

Sur la figure 2 , on a représenté un moyen de prélèvement du produit contenu dans la cellule 42 et destiné à être diffusé au moyen du dispositif selon l'invention.

De façon générale, les moyens de prélèvement comportent des bras rotatifs de prélèvement montés sur la colonne 24 et venant sélectivement se raccorder aux cellules 42, 42' sous le contrôle de l'unité centrale.

Les moyens de prélèvement comportent en outre un moyen de pompage.

Le moyen de prélèvement représenté à la figure 2 comporte un tube de raccordement 44 mettant en communication la cellule 42 avec la colonne 24 par ouverture de la valve.

Comme décrit précédemment, le disque 32 est monté à rotation sur la colonne 24.

Il serait cependant possible, en variante, de monter le disque 32 de façon fixe sur la colonne 24, le tube de raccordement 44 étant dans ce cas déplaçable angulairement par rapport à la colonne 24 sous l'action de moyens moteurs (non représentés).

Le dispositif 2 qui vient d'être décrit en référence aux figures 1 et 2 fonctionne de la façon suivante.

On insert dans l'ouverture 34 latérale pratiquée dans le boîtier 4 du dispositif de diffusion 2 selon l'invention, les disques 28, 30 et 32, dont l'ensemble des cellules 42, 42' ont été préalablement remplies de produits destinés à être diffusés, soit par le pulvérisateur 16, soit par la deuxième chambre de diffusion 10, selon la nature des produits contenus dans les cellules 42, 42'.

En fonction du programme transmis par l'unité centrale et donc des ordres de diffusion, les moyens moteurs sont activés pour que le disque contenant le produit à diffuser effectue une rotation permettant le positionnement de la cellule correspondante au niveau du moyen de prélèvement, qui se raccorde ensuite à la valve située à l'embouchure de cette cellule.

Dans le cas où le produit à diffuser est du type huiles essentielles ou éléments minéraux, un volume prédéterminé du dit produit est pompé, à partir du contenu d'une cellule portée par le deuxième ou troisième disque 30, 32, sous l'action d'une pression verticale exercée par la pompe localisée dans la colonne 24, puis conduit vers le réceptacle 20 situé dans la seconde chambre de diffusion 10.

Le produit est alors mis sous forme gazeuse, par une étape d'agitation moléculaire par exemple.

Les molécules gazeuses du produit ainsi formées se répandent dans la seconde chambre de diffusion 10, puis, sous l'action des ventilateurs 22, hors du dispositif de diffusion 2, c'est-à-dire dans l'air ambiant 18.

Au contraire, dans le cas où le produit à diffuser est constitué par des parfums naturels ou de synthèse, une quantité de produit à diffuser est prélevée et est dirigée, par aspiration au moyen de la pompe, à l'extrémité supérieure de la colonne 24. Une membrane vient alors obstruer cette extrémité supérieure, enfermant le produit à diffuser. La pression s'inverse et le produit est alors propulsé dans l'air ambiant 18 par le pulvérisateur 16.

On peut prévoir, sur le boîtier 4, un voyant lumineux qui s'éclaire afin d'indiquer à l'utilisateur que le disque 32 inséré comporte au moins une cellule vide.

L'ensemble de ces opérations mécaniques sont commandées par l'unité centrale, en fonction d'un programme de diffusion prédéterminé stocké en mémoire dans cette dernière et assurant le déroulement de séquences de diffusion en fonction de séquences d'ordres de diffusion prédéterminés stockées en mémoire dans le dispositif de diffusion 2 ou téléchargeables à distance.

Ainsi, grâce au dispositif de l'invention, il est enviseageable de créer et d'enchaîner la diffusion de différents produits. De tels produits peuvent être de même nature, auquel cas seul le moyen de diffusion adapté à la diffusion des dits produits est sollicité ; mais il est aussi possible de diffuser des produits qui ne sont pas de même nature : dans ce dernier cas, chaque moyen de diffusion spécifiquement adapté au produit considéré est actionné en fonction des séquences d'ordres de diffusion prédéterminés.

Grâce aux procédé de diffusion et dispositif de l'invention, il est ainsi possible de créer ce que l'on appelle des "architectures olfactives", formées par des diffusions successives d'au moins deux produits distincts, dès lors que certains de ces produits présentent des propriétés olfactives différentiables les unes des autres.

On peut en outre doter le dispositif selon l'invention d'un ou plusieurs systèmes annexes, ayant pour effet de combiner la diffusion des produits à une action mécanique, chimique et/ou thermique complémentaire afin de reproduire une architecture olfactive suivant des caractéritiques détectées dans l'environnement, pour tenir compte du contexte dans lequel sont sensés être diffusés les produits.

Ainsi, sur la figure 3, on a représenté un mode particulier d'installation du dispositif selon l'invention selon lequel les ordres de diffusion peuvent être téléchargés à partir d'un centre serveur dédié à cette application.

Selon cet agencement, trois dispositifs 100, 102 et 104 sont connectés respectivement à trois ordinateurs personnels 106, 108 et 110 placés en réseau.

Préférentiellement, le dispositif selon l'invention peut être intégré dans un ordinateur du type PC.

Avantageusement, les ordinateurs personnels 106, 108 et 110 sont raccordés au réseau internet 112, le serveur 114 constituant dès lors un serveur d'un site internet.

Dans ce cas, on peut envisager un fonctionnement selon lequel avec la première page transmise, par le serveur, à l'un des ordinateurs personnels 106, 108, 110, ce dernier transmet au dit ordinateur personnel des données de diffusion de produits sous la forme d'une appliquette.

On conçoit dès lors que, de manière générale, il est possible de provoquer la diffusion de produits en synchronisme avec le défilement d'images et/ou de sons,... d'un film, de radio,... en transmettant simultanément avec ces informations des ordres de diffusion d'un ou plusieurs produits correspondants.

L'installation de tels dispositifs de diffusion selon la présente invention est ainsi envisageable aussi bien dans des bâtiments d'habitation, hôtels, bureaux, magasins, salles de spectacles,... que dans des espaces clos mobiles (véhicules, navires, avions).

En particulier, l'unité centrale et le dispositif peuvent être embarqués à bord d'un véhicule ou être commandés à distance, par exemple par des signaux transmis par un téléphone mobile.

La sélection des produits à diffuser peut alors être effectuée en fonction du lieu où se trouve le véhicule dont les caractéristiques peuvent être fournies par des capteurs d'odeurs ou par un dispositif de positionnement par satellite (GPS).

De tels dispositifs peuvent également être intégrés à différents types d'appareils tels que, par exemple, montres, réveils, aspirateurs, consoles de jeux, ordinateurs,...

On notera enfin que la diffusion des produits peut être aussi effectuée en fonction de l'heure, des paramètres de l'air ambiant 18 (température, odeurs, niveau sonore,...) tels que mesurés par le capteur 46.

Le dispositif ainsi que le procédé selon l'invention peuvent également être envisagés pour la diffusion de compositions, une composition étant définie comme le mélange d'au moins deux produits distincts.

## Revendications

1. Dispositif de diffusion (2) dans l'air ambiant (18) d'un premier produit, comprenant un moyen de stockage (28, 30, 32) du premier produit à l'abri de l'air, des moyens de prélèvement (24, 44) du premier produit à partir du dit moyen de stockage (28, 30, 32), un moyen de diffusion (10, 16) du premier produit prélevé et une unité centrale de traitement raccordée aux dits moyens de prélèvement (24, 44) du premier produit et au dit moyen de diffusion (10, 16) du premier produit pour piloter ces derniers en fonction d'ordres de diffusion du premier produit, **caractérisé en ce que** le dispositif (2) comporte au moins un deuxième moyen de stockage (28, 30, 32) d'au moins un deuxième produit de nature différente du premier produit, ledit deuxième moyen de stockage (28, 30, 32) communiquant avec lesdits moyens de prélèvement (24, 44), et au moins un deuxième moyen de diffusion (10, 16), chacun des moyens de diffusion (10, 16) étant adapté à la nature du produit à diffuser.

2. Dispositif de diffusion selon la revendication 1, **caractérisé en ce que** l'un des moyens de diffusion (10, 16) de produit est un pulvérisateur (16).

3. Dispositif de diffusion selon l'une des revendications 1 et 2, **caractérisé en ce que** l'un des moyens de diffusion (10, 16) est une chambre de diffusion (10) qui communique avec l'air ambiant (18) et dans laquelle débouchent les moyens de prélèvement (24, 44).

4. Dispositif de diffusion selon la revendication 3, **caractérisé en ce que** la chambre de diffusion (10) est dotée intérieurement d'un réceptacle (20) dans lequel débouchent les moyens de prélèvement (24, 44) et communiquant avec la chambre de diffusion (10), et de moyens de ventilation (22) pilotés par l'unité centrale.

5. Dispositif de diffusion selon l'une quelconque des revendications 1 à 4, **caractérisé en ce que** les premier et deuxième moyens de stockage (28, 30, 32) comportent un ensemble de cellules (42, 42') servant chacune au stockage des premier et deuxième produits, et **en ce que** les moyens de prélèvement (24, 44) d'un des dits produits comportent un moyen de pompage.

6. Dispositif de diffusion selon la revendication 5, **caractérisé en ce que** les cellules (42, 42') sont portées par des disques (28, 30, 32) amovibles venant se monter sur une colonne (24) délimitant intérieurement un conduit (26) débouchant par l'une de ses extrémités dans le pulvérisateur (16) et par son extrémité opposée dans la chambre de diffusion (10).

7. Dispositif de diffusion selon la revendication 6, **caractérisé en ce que** les moyens de prélèvement (24, 44) comportent en outre des bras rotatifs de prélèvement montés sur la colonne (24) et venant sélectivement se raccorder aux cellules (42, 42') sous le contrôle de l'unité centrale.

8. Dispositif de diffusion selon l'une quelconque des revendications 3 à 7, **caractérisé en ce que** le pulvérisateur (16) constitue un moyen de diffusion de parfums naturels ou de synthèse et **en ce que** la chambre de diffusion (10) constitue un moyen de diffusion d'huiles essentielles ou d'éléments minéraux.

9. Dispositif de diffusion selon l'une quelconque des revendications 1 à 8, **caractérisé en ce qu'**il comporte en outre un capteur (46) destiné à fournir des données sur l'air ambiant (18), ces données étant recueillies, analysées et traitées par l'unité centrale.

10. Procédé de diffusion dans l'air ambiant (18) d'un premier produit, à l'aide d'un dispositif (2) comportant un moyen de stockage (28, 30, 32) du premier produit à l'abri de l'air, et comprenant des étapes telles qu'on le prélève à partir du dit moyen de stockage (28, 30, 32), on le conduit dans un moyen de diffusion (10, 16) et on le diffuse, ces étapes étant pilotées et contrôlées par une unité centrale, **caractérisé en ce que** le dispositif (2) comportant au moins un deuxième moyen de stockage (28, 30, 32) d'au moins un deuxième produit à diffuser de nature différente du premier produit, et au moins un deuxième moyen de diffusion (10, 16), chacun des moyens de diffusion (10, 16) étant adapté à la nature du produit à diffuser, le procédé comprend en outre une étape de sélection du produit à diffuser et une étape de sélection du moyen de diffusion (10, 16) en fonction de la nature du produit à diffuser.
